# EUROPEAN PATENT APPLICATION

(11) **EP 4 454 570 A1**
(43) Date of publication of application: **30.10.2024**
(21) Application number: 23170333.1
(22) Date of filing: 27.04.2023
(51) Int. Cl.: A61B 10/00, A61B 10/02, A61B 10/04

(54) **DEVICE AND SYSTEM FOR FINE NEEDLE ASPIRATION**

(71) Applicant: FUJIFILM Corporation, Tokyo 107-0052 (JP)
(72) Inventor: DIETER, Johannes, 96050 Bamberg (DE)
(74) Representative: Meissner Bolte Partnerschaft mbB

(57) **Abstract**

The present invention relates to a device (1), which comprises: a needle (2) defining a first passageway (P1) along the longitudinal axis thereof; a handle (3) defining a second passageway (P2) therein which extends along a longitudinal axis of the handle (3) and is in fluid communication with the first passageway (P1), the handle (3) having a first port (10) at its proximal end; and a stylet (4) extending from the first port (10) and through the first and second passageways (P1, P2). The device (1) further comprises: a sealing means (12) configured to seal the second passageway (P2) from the environment outside of the first port (10); and a second port (11) which is in fluid communication with the second passageway (P2) at a location between the sealing means (12) and the first passageway (P1).

## Description

### TECHNICAL FIELD

This invention relates to a subfield of ultrasound endoscopy (endoscopic ultrasound: EUS). Biopsy needles are used to take tissue samples. Two techniques are distinguished here: fine needle aspiration, FNA; and fine needle biopsy FNB. FNA is primarily used to aspirate fluids and/or tissues, whereas FNB is used to remove solid material.

More specifically, FNA is performed with the aid of an endoscope, called endoscopic ultrasound-guided fine-needle aspiration (EUS-FNA). Endoscopy is a medical procedure in which a flexible instrument that has a camera on its end is passed into an area of a human or animal body, such as the gastrointestinal or GI tract. Physicians, known as gastroenterologists, use endoscopy to review a patient's GI tract and may employ a variety of endoscopic procedures. Depending on what is being examined, several other types of endoscopic procedures can be used.

Endoscopic ultrasound (EUS) combines endoscopy with ultrasound or echo endoscopy to obtain details of the digestive tract, including the pancreas and surrounding abdominal organs. EUS-FNA is a type of procedure that combines steps involving imaging, tissue sampling and on occasion collection of pancreatic cyst fluid.

The EUS-FNA procedure involves the insertion of a thin, flexible, echo endoscope into the mouth, oesophagus, stomach and further into the small intestine known as the duodenum. At the tip of the echo endoscope is an ultrasound transducer. The sound waves emitted and detected by the transducer are converted into detailed images of the pancreas and surrounding abdominal oranges.

EUS-FNA simultaneously involves a procedure referred to as fine needle aspiration, FNA, which allows doctor to sample areas of interest for analysis.

FNA uses a specialised needle that can be inserted through the wall of the stomach or intestine into the pancreas. Using EUS for guidance, the physician is able to accurately visualise and direct the needle to the target area. The sound waves emitted from the ultrasound transducer yield detailed images when these waves bounce back and are analysed. In the case of a pancreatic tumour, for instance, once identified and localised on the ultrasound image, the FNA needle extends from the endoscope head. The lesion is pierced and the needle is moved back and forth several times in multiple areas of the lesion to obtain an adequate sample for analysis.

FNA samples, when collected, are then used to diagnose, stage, or even rule out the presence of disease or cancer, to determine the best course of treatment.

In FNA, a vacuum is usually created by a syringe mounted on the handle, and the liquid and/or tissue is aspirated.

What all of the needles used in the above procedures have in common, is that they are equipped with a stylet that extends from the handle to the tip of the needle for the following reasons:
- Firstly, this stylet supports the needle.
- Secondly, the stylet prevents biomaterial from unintentionally entering the needle.
- Thirdly, the stylet prevents possible damage to tissue or endoscope by the needle tip.

### BACKGROUND ART

US20180280660A1 discloses a handle configured to drive movement of a bronchoscopy instrument, the handle extending along a longitudinal axis between a proximal end and a distal end of the handle and including:
a housing having an interior surface defining an interior volume with a spring housing portion; a proximal handle member disposed at least partially within a proximal portion of the interior volume of the housing; and
a first helical cam interface, the proximal handle member configured to be coupled to a proximal end of an elongate channel extending beyond the distal end of the handle, a distal end of the elongate channel coupled to the bronchoscopy device. A distal end of the elongate channel coupled to the bronchoscopy instrument; an actuation sleeve disposed at least partially within a distal portion of the interior volume of the housing and having a proximal portion with a second helical cam interface a proximal portion having a second helical cam interface that engages the first helical cam interface, a rotary handle extending distally from the proximal portion of the actuation sleeve, and a piston handle formed by a distal surface of the actuation sleeve; and a spring having a first end secured within the spring housing portion of the housing and a second end engaged with the proximal portion of the actuation sleeve.

DE10212154A1 discloses a biopsy device, consisting of a hand piece into which a hollow biopsy needle is inserted, wherein a part of the biopsy needle projecting beyond the hand piece enables sampling an area in the tissue to be diagnosed.

US20080281223A1 discloses a biopsy needle for taking a tissue sample, a handle having a handle body defining a hollow interior with a hollow interior housing portion, and a cannula assembly having an inner cannula and an outer cannula surrounding at least a portion of the inner cannula. The inner cannula has a serpentine coil attached to a distal end of the outer cannula, and the inner cannula and the outer cannula are coupled together so that the two cannulas move together longitudinally. In addition, at least a portion of the cannula assembly is housed within the handle body.

### SUMMARY

In all known systems, the handle is designed so that the stylet must be completely removed before a syringe is connected to the stylet port for aspiration. This procedure is disadvantageous for several reasons.
- It is time consuming.
- The stylet is difficult to handle outside the instrument because it wants to deform back to its stretched initial position. As a result, placement is difficult.
- When removing the stylet, there is also a risk of fluid getting caught on the tip and splashing, which poses a risk of infection.

It is an object of the present invention to overcome the disadvantages of the state of the art, in particular it is an object to provide an FNA device and system which is easier to handle and safer when performing FNA procedures.

The object is solved by the subject matter of independent claims 1 and 14.

According to an aspect of the present invention, a device is provided. The device comprises: a needle defining a first passageway along the longitudinal axis thereof; a handle defining a second passageway therein which extends along a longitudinal axis of the handle and is in fluid communication with the first passageway, the handle having a first port at its proximal end; and a stylet extending from the first port and through the first and second passageways. The further comprises: a sealing means configured to seal the second passageway from the environment outside of the first port; and a second port, the second port in fluid communication with the second passageway at a location between the sealing means and the first passageway.

According to the invention, a suction device such as a syringe may be connected to the second port of the handle, allowing that the aspiration of the liquid can take place without first removing the stylet from the first port. The interface between the stylet and the handle is equipped with a sealing means so that the negative pressure from the suction device arrives at the distal end of the needle. This greatly facilitates handling of the device as well as allows for significantly safer FNA procedures.

### BRIEF DESCRIPTION OF THE DRAWINGS

The principle of the invention will now be more fully described by way of example with reference to the accompanying drawings. The same reference numbers may be used in different drawings to identify the same or similar elements. In certain instances, the description of well-known devices and methods are omitted so as not to obscure the description of the present invention with unnecessary detail.
Figure 1 illustrates an EUS-FNA procedure performed with a conventional FNA device.
Figure 2 shows a schematic view of a system having a device according to the invention, and a suction device.
Figure 3 shows a cross-section along the longitudinal axis of the device of Figure 2.
Figure 4 shows an enlarged section of the proximal end of the handle.
Figure 5 shows a schematic view of a system having another device according to the invention, and a suction device.

### DETAILED DESCRIPTION

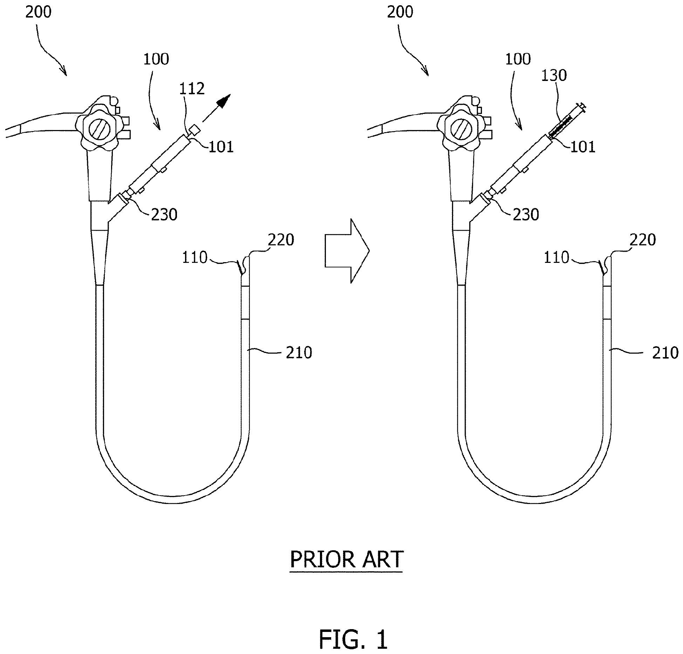

Referring to Figure 1, an EUS-FNA procedure performed with a conventional FNA device 100 is illustrated. FNA is performed with the aid of an endoscope 200. The EUS-FNA procedure involves the insertion of a thin flexible insertion 210 into the mouth, oesophagus, stomach and further into the small intestine known as the duodenum. At the tip of the echo endoscope insertion 210 is an ultrasound transducer 220. The sound waves emitted and detected by the transducer 220 are converted into detailed images of the pancreas and surrounding abdominal regions.

EUS-FNA simultaneously involves an FNA procedure, which allows physicians to sample areas of interest for analysis.

The FNA device 100 includes a specialised FNA needle 110 that can be inserted through the wall of the stomach or intestine into the pancreas, for example. Using EUS for guidance, physicians are able to accurately visualise and direct the needle to the target area. The sound waves emitted from the ultrasound transducer 220 yield detailed images when these waves bounce back and are analysed. In the case of a pancreatic tumour, for instance, once identified and localised on the ultrasound image, the FNA needle 110 may be extended out of the endoscope head.

A stylet 112 is inserted into the FNA needle 110. During puncture, the lesion is punctured with the FNA needle 110 being pulled out about 1 to several centimetres, and the stylet 112 is pushed out once inside the lesion to prevent the mucosa of the gastrointestinal tract from entering the sample. To perform FNA under negative pressure, a suction device 130 such as a syringe is connected to a port 101 formed at the proximal end of the device 100 after the stylet 112 is withdrawn and completely removed from said port 101. A vacuum is then created by the syringe 130 connected to the port 101, and the liquid and/or tissue which has been sampled is aspirated. The conventional FNA procedure is disadvantageous for several reasons. It is time consuming. The stylet is difficult to handle outside the instrument because it wants to deform back to its stretched initial position. As a result, the placement is difficult. When removing the stylet, there is also a risk of fluid getting caught on the tip and splashing, which poses a risk of infection.

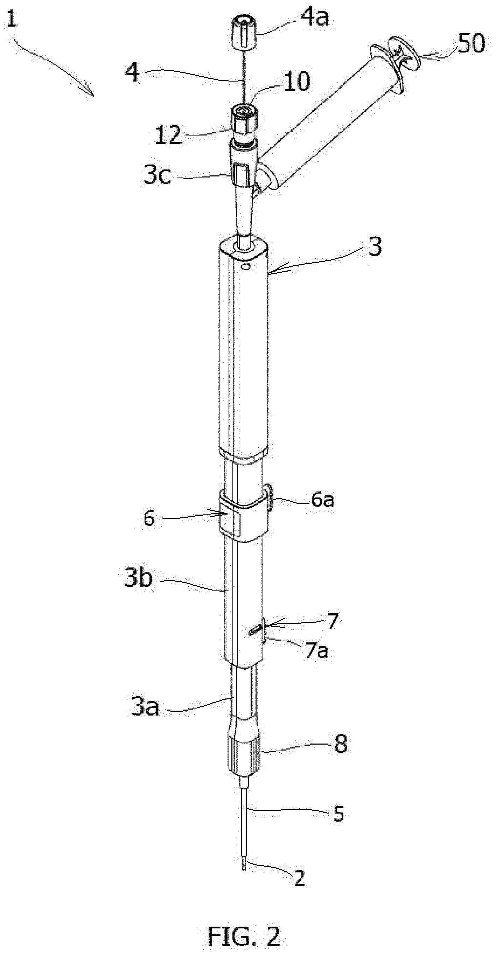

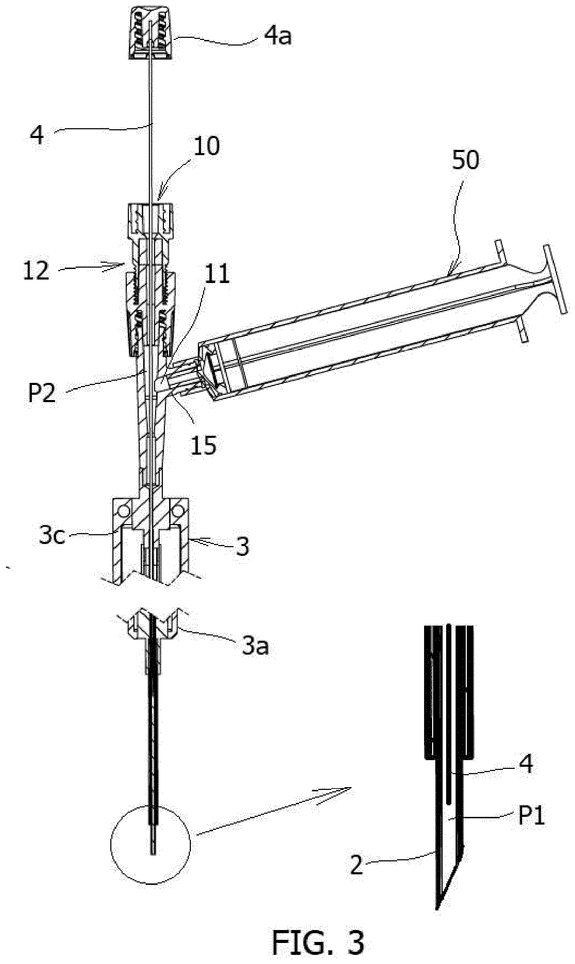

Referring to Figure 2, a device 1 according to the present invention is shown, that may overcome the aforementioned drawbacks of the conventional device. The device may be a fine needle aspiration, FNA, device; which is suitable for FNA procedures. Figure 3 shows a cross-section along the longitudinal axis of the device 1 of Figure 2.

In general, the device 1 comprises a needle 2, a handle 3, and a stylet 4.

The needle 2 is tubular and defines a first passageway P1 therein and along the longitudinal axis thereof. The diameter of the needle 2 may be 19-gauge (1.08±0.02 mm), 22-gauge (0.72±0.02 mm), or 25-gauge (0.51±0.02 mm), but other diameters may be possible depending upon end use. The needle 2 may include a side hole (not shown) which is located behind the needle tip. The side hole may be designed to anchor tissue inside the needle.

The device 1 may further include a sheath 5 to guide and protect the needle 2 therein. The sheath 5 may be a coil sheath.

The handle 3 may include a distal part 3a, a middle part 3b, and a proximal part 3c. The middle part 3a may be slidable with respect to the distal part 3a along the longitudinal axis, and the proximal part 3c may be slidable with respect to the middle part 3b along the longitudinal axis. The proximal end of the sheath 5 may be retained by the middle part 3b of the handle 3. The needle 2 may be retained by the proximal part 3c of the handle 3.

The handle 3 may include a needle length adjuster 6 and a sheath length adjuster 7. In this connection, the side of the handle may be marked with numbers indicating the extended lengths of the needle 2 and sheath 5, respectively. Each adjuster 6, 7 may include a slider with a locking knob 6a, 7a that allows the slider to be locked at any position on the side of the handle. For example, if the needle length adjuster 6, 7 is positioned and locked at number "3" marked on the side of the handle, the needle 2 will extend three centimeters when the proximal part 3c of the handle 3 is pushed down with respect to the middle part 3b. Likewise, the length of the sheath 5 extending from the tip of the endoscope insertion 210 may be adjusted by moving the sheath length adjuster 7, so that the sheath 5 is visible in the ultrasound view during the FNA procedure.

The handle 3 defines a second passageway P2 therein which extends along the longitudinal axis of the handle 3 and is in fluid communication with the first passageway P1 of the needle 2. The handle 3 may have at its distal end a connection 8 to be connected and secured to a biopsy port 230 of an endoscope 200 (see Figure 1). The connection 8 may be a thread connection or the like.

The handle 1 may also have at its proximal end a first port 10 for insertion of a stylet 4. The stylet 4, when present and inserted from the first port 10, extends through the first and second passageways P1, P2. The length of the stylet 4 extending out of the tip of the needle 2 may be adjusted by moving a cap 4a mounted on the proximal end of the stylet 4 along the longitudinal axis of the handle. The cap 4a may be threaded, as described later.

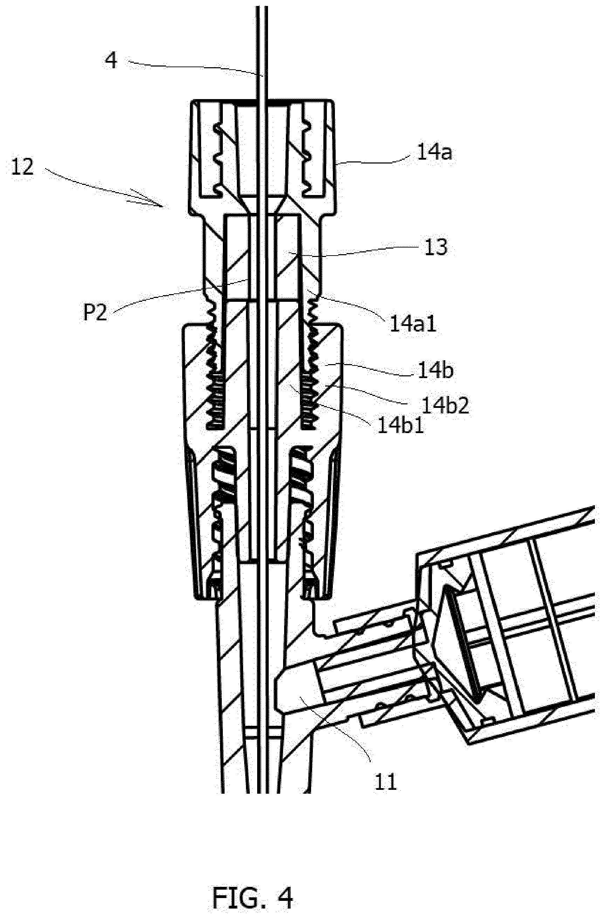

Referring to Figure 4, an enlarged section of the proximal end of the handle 3 is shown. The device 1 further comprises a second port 11, for connection to a suction device 50, and a sealing means 12.

The second port 11 is in fluid communication with the second passageway P2 at a location between the sealing means 12 and the first passageway P1. In other words, the second port 11 is located so as to allow communication with the second passageway P2 at a position distal from the sealing means 12, otherwise ambient air will be aspirated from the first port 10.

The sealing means 12 is configured to seal the second passageway P2 from the outside environment and is so located that it also seals the first port 11 from the outside environment via the second passageway P2. The sealing means 12 may be arranged at the proximal end of the second passageway P2. This increases the degree of freedom for arrangement of the second port 11. However, the sealing means 12 may be positioned more distally than in the illustrated example; in this case, the second port 11 shall also be shifted to the distal side.

The sealing means 12 may be configured to releasably seal the second passageway P2 from the outside via communication through the first port 10, such that, in the released state of the sealing means 12, the stylet 4 can be moved freely along the longitudinal axis of the handle 3 with respect to the sealing means 12. This facilitates adjustment of the position of the stylet 4 with respect to the needle 2, because during this adjustment, the sealing function by the sealing means 12 can be disengaged, allowing the stylet to move smoothly along the longitudinal axis without friction of the sealing means 12.

The sealing means 12 may include an elastic body 13 and a seal activator 14. The elastic body 13 can be tubular and may define an aperture which forms part of the second passageway P2 through which the stylet 4 passes. The elastic body 13 is formed and designed that the diameter of the aperture formed through the elastic body 13 will be reduced in the radial direction and thus close the aperture when the elastic body 13 is compressed along its longitudinal axis. The diameter of the aperture in the elastic body 13 may be smaller than that of the other parts of the second passageway P2, even when the elastic body 13 is at rest. The sides of the aperture of the elastic body 13 may be partly or fully in contact with the stylet 4 when the elastic body 13 is at rest. In any case, when the elastic body 13 is compressed along the longitudinal axis, the sides of the aperture through elastic body 13 will be pressed against the stylet 4 as the elastic body 13 expands, and a seal is thus successfully established between the elastic body 13 and the stylet 4.

Silicone may be one of suitable materials for the elastic body 13. Other elastic materials, however, may be used as long as proper sealing functionality is obtained.

The seal activator 14 is configured to compress the elastic body 13 along the longitudinal axis thereof, reducing the diameter of the aperture and thus closing said aperture of the elastic body 13. In one example, the seal activator 14 has a pair of engaging parts 14a, 14b that engage each other along the longitudinal axis to thus provide a compressive force to the elastic body 13 when interposed there-between. The engaging parts 14a and 14b are hard shell-components which are coupled with each other by, e.g., a threaded engagement. The elastic body 13 or tube section is compressed along its longitudinal axis when the respective threads of the engaging parts 14a, 14b are engaged with each other and tightened. Accordingly, the pair of engaging parts 14a, 14b can be releasably and threadably tightened together. When the first engaging part 14a on the proximal side is untightened or removed from engagement from the second engaging part 14b on the distal side, the elastic body 13 will be released from compression, or experience a lower compressive force, and the seal between the elastic body 13 and the stylet 4 may then be broken. On the contrary, when the first engaging part 14a is thread-tightened into engagement with the second engaging part 14b, the elastic body 13 is compressed, or experiences a higher compressive force, and will be squeezed against the stylet 4, closing the aperture in the elastic body 13. This creates a seal between the elastic body 13 and the stylet 4.

In the illustrated example, the first engaging part 14a has an annular wall 14a1 that defines an opening toward the distal end. The annular wall 14a1 and opening define a receptacle for the elastic body 13. The outer surface of the annular wall 14a1 is preferably threaded. On the other hand, the second engaging part 14b has a plug 14b1 to be inserted into the opening defined by annular wall 14a1 of the first engaging part 14a. The second engaging part 14b further has an annular wall 14b2 that extends around the plug 14b defining an annular groove there-between. The inner surface of the annular wall 14b2 is also preferably threaded so as to allow engagement with the threaded outer surface of the annular wall 14a1. It is preferable that the outer surface of the elastic body 13 is in contact with, or substantially in contact with, the inside of the annular wall 14a1 of the first engaging part 14a even under the uncompressed state of the elastic body 13. As a result, when the elastic body 13 is compressed, the elastic body 13 expands only in the direction of closing the aperture formed therein.

Other means than the threads for compressing the elastic body 13 between the first and second engaging parts 14a, 14b are also possible. For example, the first and second engaging parts 14a, 14b may be bayonet-connected together. Alternatively, a latch may cause the first engaging part 14a to detachably engage the second engaging part 14b with the elastic body 13 being compressed.

The second engaging part 14b on the distal side may be secured to the distal part 3c of the handle 3 by means of thread, or rib-to-groove or rib-to-rib engagement. Alternatively, the second part 14b may be integrally formed or moulded with the handle body, i.e., as integral part of the handle 3. The latter is advantageous, because fewer individual parts are required.

The proximal end of the first engaging part 14a may be threaded so that the cap 4a of the stylet 4 can be fitted thereon and connected therewith. This prevents the stylet 4 from detaching from the handle 3 in a shipping package, preferably in a sterile package. It also ensures that the stylet 4 remains within the needle 2 when the needle 2 is inserted into the endoscope channel.

Referring again to Figure 3, the second port 11 may include a connector 15 which extends from the side of the handle 3. The suction device 50 may comprise a syringe which may be connected to the second port 11 via the connector 15. As shown, the connector 15 may be inclined upward toward its tip. Accordingly, the suction device will be mounted to the handle 3 in an upward slant (Y-arrangement). Good operability may then be expected when applying suction via the suction device 50. Alternatively, the connector 15 may protrude at right angle from the side of the handle 3. In this case, the suction device 50 will be mounted at a right angle with respect to the handle 3 (T-arrangement).

The connector 15 may be a luer-lock connector or luer taper connector. This allows safe and secured connection between the suction device 50 and the second port 11. Preferably, the suction device 50 comprises a syringe with a Luer-Lock fitting.

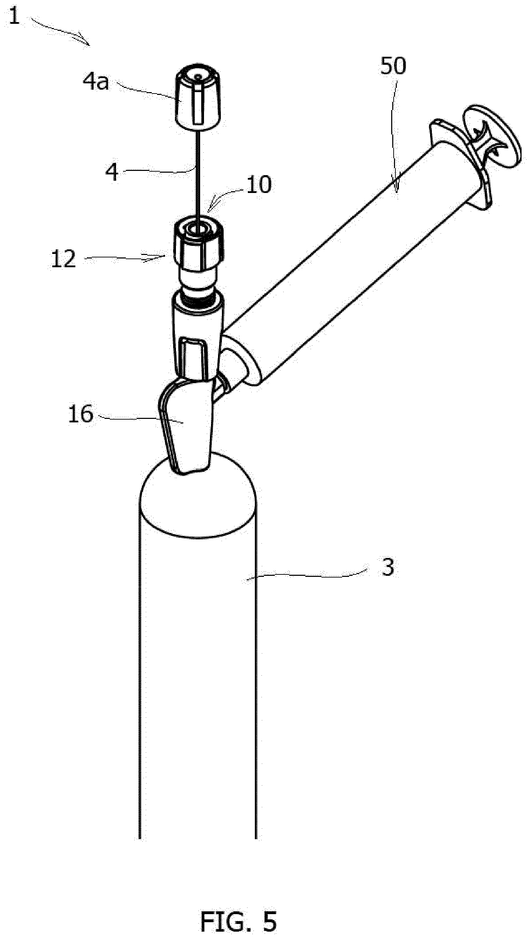

As shown in Figure 5, a grip 16 may be provided on the handle 3 to improve operability during FNA. Preferably, the grip 16 and the second port 11 are opposed to each other with the longitudinal axis of the handle 3 interposed there-between. In other words, the grip 16 extends away from the connector 15. The grip 16 may be gripped by a user during FNA with the suction device 50.

The device 1 according to the invention is easier and safer to use than conventional FNA devices, because sample tissue or liquid can be collected by the suction device 50 without having to remove the stylet 4 from the first port 10. More specifically, after adjusting the position of the stylet 4 within the needle 2, the sealing means 12 can be activated to seal the second passageway P2 from the environment outside of the first port 10. After puncturing a target area of a human or animal body with the needle 2, the second and first passageways P2, P1 are depressurised with the suction device 50, allowing liquid or tissue to be drawn from the target area through the clearance between the needle 2 and the stylet 4, then through the second passageway P2 and further into the suction device 50 through the second port 11. Sufficient clearance between the stylet 4 and the inner diameter of the FNA needle 2 is preferable to allow sample liquid and/or tissue to be aspirated through the clearance.

### LIST OF REFERENCE NUMERALS

- 1: Device
- 2: Needle
- 3: Handle
- 4: Stylet
- 4a: Cap
- 5: Sheath
- 6: Needle length adjuster
- 7: Sheath length adjuster
- 8: Connection
- 10: First port
- 11: Second port
- 12: Sealing means
- 13: Elastic body
- 14a: Engaging part
- 14b: Engaging part
- 15: Connector
- 16: Grip
- P1: First passageway
- P2: Second passageway
- 50: Suction device

## Claims

1. A device (1), comprising:
a needle (2) defining a first passageway (P1) along the longitudinal axis thereof;
a handle (3) defining a second passageway (P2) therein which extends along a longitudinal axis of the handle (3) and is in fluid communication with the first passageway (P1), the handle (3) having a first port (10) at its proximal end; and
a stylet (4) extending from the first port (10) and through the first and second passageways (P1, P2);
the device (1) further comprising:
a sealing means (12) configured to seal the second passageway (P2) from the environment outside of the first port (10); and
a second port (11) which is in fluid communication with the second passageway (P2) at a location between the sealing means (12) and the first passageway (P1).

2. The device (1) according to claim 1, wherein the sealing means (12) is arranged at the proximal end of the second passageway (P2).

3. The device (1) according to claim 1 or 2, wherein the sealing means (12) is configured to releasably seal the second passageway (P2) from the environment outside of the first port (10), such that, in the released state of the sealing means (12), the stylet (4) can move freely along the longitudinal axis of the handle (3) with respect to the sealing means (12).

4. The device (1) according to any one of claims 1 to 3, wherein the sealing means (12) includes:
an elastic body (13) defining an aperture which forms a part of the second passageway (P2) through which the stylet (4) passes; and
a seal activator (14) configured to compress the elastic body (13) along the longitudinal axis of the handle (3), and in so doing reducing the diameter of, or closing, said aperture of the elastic body (13).

5. The device (1) according to claim 4, wherein the seal activator (14) has a pair of engaging parts (14a, 14b) that engage each other in the longitudinal axis to enable a compressive force to be applied to the elastic body (13) along the longitudinal axis.

6. The device (1) according to claim 4 or 5, wherein the elastic body (13) is made of silicone.

7. The device (1) according to any one of claims 4 to 6, further comprising a cap (4a) at a proximal end of the stylet,
wherein the cap (4a) is detachably fitted to the seal activator (14), preferably by means of thread.

8. The device (1) according to any one of claims 1 to 7, wherein there is a clearance between the stylet (4) and the needle (2).

9. The device (1) according to any one of claims 1 to 8, wherein the second port (11) includes a connector (15) which extends from a side of the handle (3).

10. The device (1) according to claim 9, wherein the connector (15) extends upwardly toward its tip.

11. The device (1) according to claim 9 or 10, wherein the connector (15) is a luer-lock connector.

12. The device (1) according to any one of claims 1 to 11, further comprising a grip (16), preferably the grip (16) and the second port (11) being opposed each other with the longitudinal axis of the handle (3) interposed therebetween.

13. The device (1) according to any one of claims 1 to 12, which is a fine needle aspiration device.

14. A system comprising:
a device (1) according to any one of claims 1 to 13; and
a suction device (50) attached to the second port (11) of the device (1).

15. The system according to claim 14,
wherein the suction device (50) comprises a syringe, more preferably a luer-lock syringe.
